Europäisches Patentamt

European Patent Office

Office européen des brevets

(11) Publication number: **0 305 206 B1**

# EUROPEAN PATENT SPECIFICATION

(45) Date of publication of patent specification: **14.10.92**   (51) Int. Cl.⁵: **G01N 25/52**, G01N 33/28

(21) Application number: **88307942.8**

(22) Date of filing: **26.08.88**

(54) **Apparatus for measuring flash point of petroleum intermediate fraction and method for controlling flash point.**

(30) Priority: **27.08.87 JP 211247/87**

(43) Date of publication of application:
**01.03.89 Bulletin 89/09**

(45) Publication of the grant of the patent:
**14.10.92 Bulletin 92/42**

(84) Designated Contracting States:
**DE FR GB NL**

(56) References cited:
**DE-B- 2 723 157**
**US-A- 2 939 312**
**US-A- 3 440 863**
**US-A- 3 748 894**
**US-A- 4 092 847**

**PATENT ABSTRACTS OF JAPAN, vol. 9, no. 273 (P-401)(1996), 30 October1985; &JP-A-60119453**

(73) Proprietor: **Nippon Oil Co., Ltd.**
**3-12, Nishishinbashi 1-chome Minato-ku Tokyo 105(JP)**

(72) Inventor: **Saito, Naohide**
**3-2-10-235, Sasaguchi**
**Niigata-shi Niigata-ken(JP)**
Inventor: **Inomata, Kentaro**
**4-14-27, Botanyama**
**Niigata-shi Niigata-ken(JP)**
Inventor: **Shibuya, Shohei**
**3-4-29, Botanyama**
**Niigata-shi Niigata-ken(JP)**

(74) Representative: **Slight, Geoffrey Charles et al**
**Graham Watt & Co. Riverhead**
**Sevenoaks Kent TN13 2BN(GB)**

## Description

### 1. Field of the Invention

The present invention relates to an on-line apparatus for measuring the flash point of a petroleum intermediate fraction such as kerosene during the distillation of a crude oil, and it also relates to a method for controlling the flash point of the petroleum intermediate fraction by the utilization of the measured value obtained by the above-mentioned measuring apparatus.

### 2. Description of the Prior Art

With regard to components of petroleum such as a gasoline fraction and an intermidiate fraction such as kerosene, their boiling points are continuously distributed, and when these components are extracted from crude oil by distillation, kerosene is stripped or heated with steam to vaporize volatile components therefrom, and these volatile components are then delivered into a gasoline fraction, whereby the flash point of kerosene is adjusted.

In this case, if the flash point of kerosene is measured by an on-line system, the amount of the steam, for example, used for heating or stripping the kerosene can be regulated on the basis of the measured value, so that the flash point can be stably maintained. In contrast, in an off-line system, in general, kerosene is first sampled and its flash point is then measured directly, and the amount of the steam is controlled with the aid of operational experience making reference to the measured value. Accordingly, the accurate control of the amount of steam to be used is difficult owing to time lag and the like. It is thus necessary to make large allowances for safe control, which obstructs efficient mass production of kerosene.

DE-A-2 723 157 discloses a method for indirectly measuring the flash point on an off-line system via the total carbon concentration in the gas/vapor phase. The concentration is determined by means of the flame ionization detector (FID). The flash point is calculated from an equation which is linear in regard to the concentration.

In a conventional known method for directly measuring a flash point by an on-line system, a certain amount of a liquid to be measured is first sampled, and a spark is emitted through the gaseous phase above the liquid during heating in order to ignite the gas. The flash point is established and measured by detecting the generation of sound or light.

US-A-3,440,863 discloses an improved on-line system apparatus for directly determining the flash point of a liquid having a flash point higher than about 85°C. The apparatus utilizes a flash chamber having liquid and vapor chambers therein equipped with improved heating means. It also has a new device suitable for detecting high flash points consisting of a housing with a diaphragm. In methods for directly measuring the flash point in an on-line system, the flash point of the liquid is measured at certain time intervals, a technique which is substantially similar to off-line systems.

US-A-4,092,847 discloses a method for indirectly measuring the flash point of an oil product on an on-line system in which at least the specific gravity and viscosity of the product are measured and the flash point is then calculated from the empirical equation relating these values to the flash point.

In this case, needless to say, an on-line device for measuring the specific gravity or viscosity is required, and the measurement accuracy of the flash point depends upon the performance of the measuring device.

As discussed above, in a crude oil distillation process, any convenient and reliable device and method for detecting the flash point of the petroleum intermediate fraction such as kerosene by a continuous on-line system has not been heretofore established.

### SUMMARY OF THE INVENTION

The present invention intends to solve the above-mentioned problems. An object of the present invention is to provide an apparatus for measuring the flash point of a petroleum intermediate fraction simply and reliably by a continuous on-line system, and another object of the present invention is to provide a method for controlling the flash point of the intermediate fraction efficiently.

According to one aspect of the present invention, the flash point of an intermediate fraction such as kerosene is continuously measured in the distillation process of petroleum in an on-line system with the aid of a signal obtained by dividing the difference between gas concentrations of the intermediate fraction at two predetermined temperatures by the difference between the predetermined temperatures. The thus measured flash point of the intermediate fraction can be used to heighten the extraction efficiency of volatile components for gasoline and the like from the intermediate fraction.

The inventors of the present application have researched as to whether or not a flash point of a liquid can be determined by heating the liquid and then measuring the rate of change in the amount or concentration of an inflammable gas above the liquid with respect to a temperature. As a result, it has been found that there is a definite interrelation between the flash point and the rate of change in the amount or concentration of the gas to the temperature. On the basis of this knowledge, the present invention which is quite novel has been achieved.

Describing in detail, a difference between amounts or concentrations of the gas at two positions having the predetermined temperatures is employed to obtain the rate of change to the temperature. Therefore, it is not always necessary to measure the absolute amount of the gas.

The first aspect of the present invention is directed to an on-line apparatus for indirectly measuring the flash point of a petroleum intermediate fraction during distillation of crude oil, characterized by a first device for continuously measuring a first gas concentration at a first predetermined temperature not higher than 40°C of the intermediate fraction to provide a signal indicating the first gas concentration, a second device for continuously measuring a second gas concentration at a second predetermined temperature lower than the first predetermined temperature e.g. 10C° lower, to provide a signal indicating the second gas concentration, and an arithmetical unit for dividing the difference ($\Delta C$) between the first and second gas concentrations by the difference ($\Delta T$) between the first and second predetermined temperatures to provide a signal of the quotient (K), and carrying out an arithmetical treatment in accordance with the equation: Flash point = 57-8.8K to obtain a signal corresponding to the flash point of the intermediate fraction from the value of K determined from $\Delta C$ and $\Delta T$.

The second aspect of the present invention is directed to a method for controlling the flash point of a petroleum intermidiate fraction during distillation of crude oil, characterized by the steps of continuously measuring the concentration of a first gas at a first predetermined temperature not higher than 40°C of the intermediate fraction, continuously measuring the concentration of a second gas at a second predetermined temperature lower than the first predetermined temperature e.g. 10C° lower, dividing the difference ($\Delta C$) between the first and second gas concentrations by the difference ($\Delta T$) between the first and second predetermined temperatures to provide a signal of the quotient (K), carrying out an arithmetical treatment of the signal (K) in accordance with the equation: Flash point = 57-8.8K to provide a signal indicative of the flash point and utilizing this signal to control the feed of a heat source such as stripping steam or oil for heating the fraction drawn from the bottom of the stripper or the fractionator for the intermediate fraction.

One way of carrying out the present invention in both its method and apparatus aspects will now be described in detail by way of example with reference to drawings in which:

FIG. 1 is a graph illustrating the relation between rates of change in gas concentrations at oil temperatures of 30 to 40°C and flash points of the oil; and

FIG. 2 is a flow chart illustrating one embodiment of apparatus of the present invention.

In these drawings,

1       Crude oil distillation column
2       Kerosene stripper
3       Gas oil stripper
4       First cooler for setting temperature
5       Second cooler for setting temperature
6       First pot for gaseous sample
7       Second pot for gaseous sample
8       Kerosene extraction line
9       Air line
10     Kerosene sample line
11     Gaseous sample line
12     Gaseous sample line
13     First detector of gas amount
14     Second detector of gas amount
15     Arithmetical unit
16     Steam control valve

## DETAILED DESCRIPTION WITH REFERENCE TO DRAWINGS

Now the present invention will be described taking kerosene as one example of a petroleum intermediate fraction.

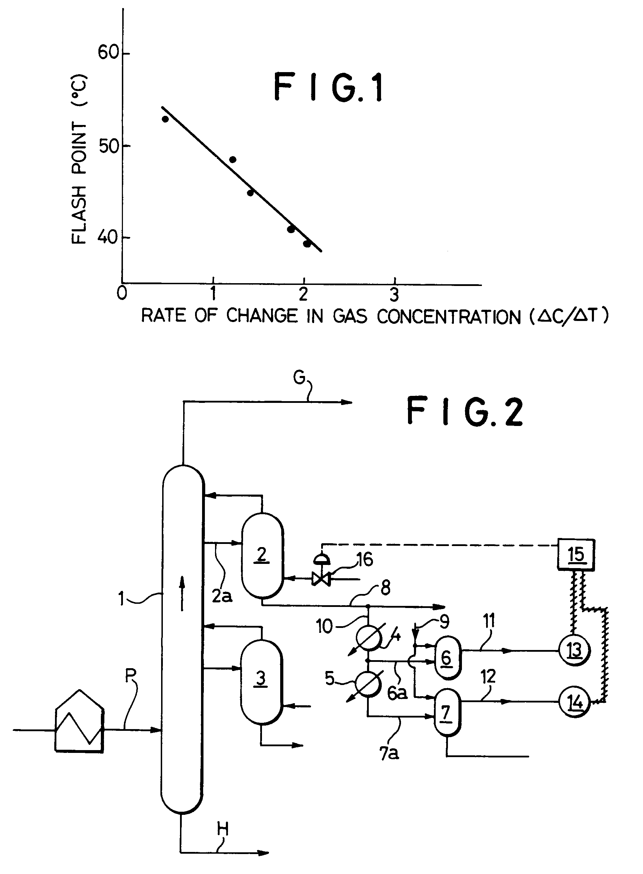

For various kerosene samples, gas concentrations in gaseous phases maintained at 30°C and 40°C were measured, and their rates of change K were then calculated. On the other hand, flash points of these kerosene samples were measured in a conventional manner. The interrelation between the rates of change and the flash points was exhibited as in Fig. 1. From the results in Fig. 1, the following formula (1) was obtained:

Flash point = 57 - 8.8K     (1)

wherein K = $\Delta C/\Delta T$, where $\Delta C$ is the difference between measured concentrations and $\Delta T$ is the difference between measured temperatures.

Each temperature to be measured may be lower than the above-mentioned levels, but when it is too low, the amount of the vaporized gas is too small to measure its concentration.

The gas concentration (gas amount) was determined by first bringing the gas of the kerosene sample into contact with a coil-like platinum catalyst which was being heated, in order to burn it, and measuring the quantity of heat generated at this time.

The platinum coil and another similar platinum coil which had not been heated were used in the form of a pair to constitute a Wheatstone bridge circuit, and when the temperature of the coil was raised by the generated heat, electric resistance of the coil correspondingly changed. From a change in the voltage of the coil changed its electric resistance, the gas amount or concentration was determined.

Two measuring devices of this type were simultaneously employed, and the gas concentration in the gaseous phase of the kerosene sample maintained at 30°C was measured by the use of one detector means (the Wheatstone bridge circuit). On the other hand, the gas concentration in the gaseous phase of the kerosene sample maintained at 40°C was measured by the other detector means in order to obtain basic data for the above-mentioned formula (1).

Referring now to Fig. 2, a crude oil was fed to a distillation column 1 as indicated by an arrow P, and the crude oil was then distilled in the column 1. Afterward, gas oil for gasoline was extracted from the top of the distillation column 1 as indicated by an arrow G and residual oil containing heavy oil, pitch and the like was discharged from the bottom thereof as indicated by an arrow H. At an upper position in the middle portion of the distillation column 1, there was provided a kerosene stripper 2. Kerosene was delivered to this kerosene stripper 2 through a kerosene pipe 2a, and the kerosene was then extracted through a kerosene extraction line 8. In the distillation column, under the kerosene stripper 2, a gas oil stripper 3 was disposed.

A kerosene sampling line 10 was connected to the extraction line 8, and on the sampling line 10, a first cooler 4 and a second cooler 5 for setting temperatures of the kerosene portions to predetermined levels were mounted. The kerosene portion which had been set to 40°C by means of the first cooler 4 was fed via a line 6a to a first pot 6 for the gaseous sample, and the kerosene portion which had been set to 30°C by means of the second cooler 5 was fed via a line 7a to a second pot 7 for the gaseous sample. To the first and second pots 6 and 7, air was fed through an air line 9, and gases generated in both the pots 6 and 7 were forwarded to a first gas amount detector 13 and a second gas amount detector 14 via gaseous sampling lines 11 and 12, respectively. That is, the gas generated from the kerosene set to 40°C was delivered to the first gas amount detector 13, and the gas generated from the kerosene set to 30°C was delivered to the second gas amount detector 14.

As described above, the first and second gas detectors 13, 14 were the Wheatstone bridge circuits each comprising a platinum catalyst which was being heated and another similar platinum catalyst which was not heated. These detectors 13, 14 detected the gas concentrations, and their data was inputted into an arithmetical unit 15. The above detection of the gas concentrations was continuously carried out by an on-line system. In the arithmetical unit 15, the difference between both the gas concentrations indicated by the data signals of the gas concentrations delivered from the first and second gas detectors 13, 14 was divided by the difference between both the predetermined temperatures (30°C and 40°C) in order to obtain a signal (see Fig. 1). This signal was subjected to arithmetical treatment by the arithmetical unit 15 in order to provide another signal corresponding to the flash point of the kerosene in the kerosene stripper 2. Furthermore, this output corresponding to the flash point of the kerosene was forwarded to a steam control valve 16 in order to control the feed of steam for heating the fraction drawn from the bottom of the kerosene stripper 2, so that the flash point of the kerosene was controlled.

The apparatus and the controlling method described above are applicable to control the feed of the heat source for fractionators other than a kerosene stripper. In the above embodiment, how to control the flash point of kerosene has been described, but the present invention can also be applied to the flash point control of gas oil and other fractions.

**Claims**

1.  An on-line apparatus for indirectly measuring the flash point of a petroleum intermediate fraction during distillation of crude oil, characterized by a first device (14) for continuously measuring a first gas concentration at a first predetermined temperature not higher than 40°C of the intermediate fraction to provide a signal indicating the first gas concentration, a second device (13) for continuously measuring a second gas concentration at a second predetermined temperature lower than the first predetermined temperature, e.g. 10C° lower, of the said intermediate fraction, to provide a signal indicating the second gas concentration, and an arithmetical unit (15) for dividing the difference $\Delta C$ between the first and second gas concentrations by the difference $\Delta T$ between the first and second predetermined temperatures to provide a signal of the quotient K, and carrying out an arithmetical treatment in accordance with the equation: Flash point = 57-8.8K to obtain a signal corresponding to the flash point of the intermediate fraction from the value of K determined from $\Delta C$ and $\Delta T$.

2.  A method for controlling the flash point of a petroleum intermidiate fraction during distillation of crude oil, characterized by the steps of continuously measuring the concentration of a first gas at a first predetermined temperature not higher than 40°C of the intermediate fraction, continuously measuring the concentration of a second gas at a second predetermined temperature lower than the first predetermined temperature e.g. 10C° lower, dividing the difference ΔC between the first and second gas concentrations by the difference ΔT between the first and second predetermined temperatures to provide a signal of the quotient K, carrying out an arithmetical treatment of the signal K in accordance with the equation: Flash point = 57-8.8K to provide a signal indicative of the flash point and utilizing this signal to control the feed of a heat source such as stripping steam or oil for heating the fraction drawn from the bottom of the stripper or the fractionator for the intermediate fraction.

**Patentansprüche**

1. On-Line-Vorrichtung zur indirekten Messung des Flammpunktes einer Erdölzwischenfraktion während der Destillation von Rohöl, **gekennzeichnet** durch eine erste Einrichtung (14) zum kontinuierlichen Messen einer ersten Gaskonzentration der Zwischenfraktion bei einer ersten vorbestimmten Temperatur von nicht höher als 40°C, um ein die erste Gaskonzentration wiedergebendes Signal bereitzustellen, eine zweite Einrichtung (13) zum kontinuierlichen Messen einer zweiten Gaskonzentration der Zwischenfraktion bei einer zweiten vorbestimmten Temperatur, welche niedriger als die erste vorbestimmte Temperatur, beispielsweise um 10°C niedriger ist, um ein die zweite Gaskonzentrationwiedergebendes Signal bereitzustellen, und eine Recheneinheit (15) zur Division der Differenz ΔC zwischen den ersten und zweiten Gaskonzentrationen durch die Differenz ΔT zwischen den ersten und zweiten vorbestimmten Temperaturen, um ein Signal des Quotienten K bereitzustellen, und zur Ausführung einer Rechenverarbeitung nach Maßgabe der folgenden Gleichung: Flammpunkt = 57-8,8K, um ein dem Flammpunkt der Zwischenfraktion entsprechendes Signal von dem Wert von K bereitzustellen, welcher aus ΔC und ΔT bestimmt ist.

2. Verfahren zum Kontrollieren des Flammpunktes einer Erdölzwischenfraktion während der Destillation von Rohöl **gekennzeichnet,** durch die Schritte, gemäß denen kontinuierlich die Konzentration eines ersten Gases der Zwischenfraktion bei einer ersten vorbestimmten Temperatur von nicht höher als 40°C gemessen wird, die Konzentration eines zweiten Gases bei einer zweiten vorbestimmten Temperatur kontinuierlich gemessen wird, welche niedriger als die erste vorbestimmte Temperatur, beispielsweise um 10°C niedriger ist, Dividieren der Differenz ΔC zwischen den ersten und zweiten Gaskonzentrationen durch die Differenz ΔT zwischen den ersten und zweiten vorbestimmten Temperaturen, um ein Signal des Quotienten K bereitzustellen, Ausführen einer Rechenverarbeitung des Signals K nach Maßgabe der folgenden Gleichung: Flammpunkt = 57-8,8K, um ein den Flammpunkt wiedergebendes Signal bereitzustellen, und Nutzen dieses Signals zur Steuerung der Versorgung einer Wärmequelle, wie eines Stripperdampfes oder Öls zum Erwärmen der vom Boden des Strippers oder der Fraktioniereinrichtung für die Zwischenfraktion abgezogenen Fraktion.

**Revendications**

1. Appareil en "liaison directe" (on-line) destiné à mesurer de façon indirecte le point d'inflammation d'une fraction intermédiaire de pétrole pendant la distillation du pétrole brut, caractérisé par un premier dispositif (14) destiné à mesurer en continu une première concentration de gaz à une première température prédéterminée non supérieure à 40°C de la fraction intermédiaire pour fournir un signal indiquant une première concentration de gaz, un second dispositif (13) destiné à mesurer en continu une seconde concentration de gaz à une seconde température prédéterminée inférieure à la première température prédéterminée, par exemple inférieure de 10°C, de cette fraction intermédiaire pour fournir un signal indiquant la seconde concentration de gaz et une unité arithmétique (15) destinée à diviser la différence ΔC entre les première et seconde concentrations de gaz par la différence ΔT entre les première et seconde températures prédéterminées pour fournir un signal du quotient K et effectuer un traitement arithmétique selon l'équation : point d'inflammation = 57 - 8,8K pour obtenir un signal correspondant au point d'inflammation de la fraction intermédiaire à partir de la valeur de K déterminée à partir de ΔC et de ΔT.

2. Procédé destiné à contrôler le point d'inflammation d'une fraction intermédiaire de pétrole pendant la distillation du pétrole brut, caractérisé par les étapes consistant à mesurer en continu la concentration d'un premier gaz à une première température prédéterminée non supérieure à 40°C de la fraction intermédiaire, à mesurer en continu la concentration d'un second gaz à une seconde température prédéterminée inférieure à la première température prédéterminée, par exemple inférieure de 10°C, à diviser la différence ΔC entre les première et seconde concentrations de gaz par la différence ΔT entre les première et seconde températures prédéterminées pour fournir un signal du quotient K, à réaliser un traitement arithmétique du signal K selon l'équation : point d'inflammation = 57 - 8,8K pour fournir un signal indicatif du point d'inflammation et utiliser ce signal pour réguler la charge d'une source de chaleur telle que de la vapeur de strippage ou du pétrole pour chauffer la fraction prélevée du fond du strippeur ou de la colonne de fractionnement pour la fraction intermédiaire.

F I G.1

FLASH POINT (°C)

60

50

40

0    1    2    3

RATE OF CHANGE IN GAS CONCENTRATION (ΔC/ΔT)

F I G.2